# EUROPEAN PATENT APPLICATION

(11) **EP 4 591 860 A1**
(43) Date of publication of application: **30.07.2025**
(21) Application number: 25153624.9
(22) Date of filing: 23.01.2025
(51) Int. Cl.: A61K 31/197, A61K 31/728, A61K 9/00, A61K 47/36, A61P 27/02

(54) **OPHTHALMIC PHARMACEUTICAL COMPOSITION FOR THE PREVENTION AND TREATMENT OF DRY EYE SYNDROME**

(30) Priority: 23.01.2024 IT 202400001167
(71) Applicant: FB Vision S.p.A., 63074 San Benedetto del Tronto (AP) (IT)
(72) Inventor: CARBONI, Paolo, 63074 SAN BENEDETTO DEL TRONTO AP (IT); LATEGANO, Paolo, 63074 SAN BENEDETTO DEL TRONTO AP (IT); GITTI, Gian Luca, 00127 ROMA (IT); IPAVEC, Valeria, 00148 ROMA (IT); SCAPAGNINI, Giovanni, 95131 CATANIA (IT)
(74) Representative: Modiano, Micaela Nadia

(57) **Abstract**

An ophthalmic pharmaceutical composition comprising hyaluronic acid and a compound useful in the modulation of nervous transmission of the painful type for use in the prevention or chronic treatment of dry eye and/or keratoconjunctivitis sicca. The composition can be formulated as eye drops, gel, or artificial tears to be instilled in the eye.

## Description

### FIELD OF THE INVENTION

The present invention relates to an ophthalmic pharmaceutical composition, preferably in the form of eye drops, for the prevention and treatment of eye diseases with an inflammatory component such as dry eye syndrome.

The invention originates in the ophthalmic field, specifically in the field of drugs for treating ocular diseases or disorders of the surface of the eye.

In particular, the present invention relates to an ophthalmic composition based on two selected active ingredients whose combination is suitable to prevent and treat diseases of the eye surface with an inflammatory component, for example dry eye and/or keratoconjunctivitis sicca.

### STATE OF THE ART

Dry eye is a multifactorial disease of the ocular surface characterized by a loss of lacrimal film homeostasis, and accompanied by ocular symptoms, in which lacrimal film instability and hyperosmolarity, ocular surface inflammation and damage and neurosensory abnormalities play an etiological role.

This ocular disease, as well as keratoconjunctivitis sicca, causes increased inflammation of the ocular surface, lesions and neurosensory abnormalities, with potential damage to the ocular surface, symptoms of discomfort, visual disturbances and, in moderate to severe cases, limitations in daily activities.

Dry eye syndrome and keratoconjunctivitis sicca are widespread eye diseases with a high incidence in the population of industrialized countries. It is estimated that dry eye syndrome affects about 20% of the population of the European Union and the United States of America.

In both ocular pathologies there is a dysfunction of the ocular structures that produce and regulate the components of the lacrimal film, including the lacrimal glands, the meibomian glands, the cornea and the conjunctiva. These dysfunctions cause a qualitative and quantitative tear deficiency with consequent hyperosmolarity and instability of the lacrimal film.

It is well known that the lacrimal film is found on the cornea, has a thickness of about 2-5 µm and is composed of the lipid, aqueous and mucin components or layers. The lipid component is the most superficial layer, is produced by the meibomian glands of the eyelids and reduces tear evaporation.

The aqueous component is the intermediate and thickest layer of the lacrimal film. This layer is produced by the lacrimal glands, located in the eye sockets, and by the accessory lacrimal glands, in particular Krause's and Wolfring's glands, which are located in the conjunctiva of the eye.

The basal component is the innermost layer of the lacrimal film, is composed of mucins or glycoproteins, and is produced mainly by the cells of the conjunctival calyx. Mucins improve the diffusion of the lacrimal film on the corneal epithelium by regulating surface tension.

The etiologies that can contribute to the development of dry eye syndrome are various and in many cases can be multifactorial. Among the most common factors we include local ones, for example: a) skin diseases of or around the eyelids, such as rosacea or eczema, dysfunction or inadequate or altered secretions of the meibomian glands; b) ocular instillation of topical medications, for example to treat glaucoma, or eye drops containing preservatives; c) systemic diseases, for example Sjogren's syndrome and autoimmune or connective tissue disorders such as rheumatoid arthritis and lupus, thyroid diseases; d) iatrogenic causes, such as the use of systemic drugs such as antihistamines, antihypertensives, anxiolytics, diuretics, systemic hormones, non-steroidal anti-inflammatory drugs, corticosteroids, anticholinergics, isotretinoin and antidepressants, or even ophthalmic surgery, including refractive and cataract surgery, keratoplasty and eyelid surgery; f) ocular allergies and environmental factors such as exposure to irritants, cigarette smoke, smog, low humidity, the use of electronic device screens; g) chemical or thermal burns affecting the conjunctiva; h) decreased corneal sensitivity due to prolonged use of contact lenses, herpes virus infections; i) hyper- or hypovitaminosis, for example vitamin A deficiency.

The classification of dry eye syndrome from a pathological point of view involves division into two main types: aqueous and evaporative deficiency, and the possible presence of both.

Aqueous lacrimal deficiency is characterized by inadequate tear production, for example due to Sjogren's syndrome, diseases, inflammations and/or dysfunctions of the lacrimal gland, obstruction of the lacrimal gland and use of systemic or local drugs.

Evaporative dry eye is characterized by increased evaporation of the lacrimal film and a deficiency of the lipid part of the lacrimal film. In this pathological form there is a physiological production of tears; however, the altered composition of the tears causes excessive evaporation. This alteration is frequently caused by a dysfunction of the meibomian glands that line the eyelid margins and secrete fatty acids and lipid substances that make up the lipid layer of the lacrimal film and reduce the evaporation of the aqueous component of tears. Other causes of evaporative dry eye are constituted by insufficient blinking, which typically manifests itself in the case of a low frequency of eyelid closure or incomplete closure, disorders of eyelid opening, vitamin A deficiency, the use of contact lenses and environmental factors, in particular low humidity and high air flow, such as occurs in the pressurized environment of aircraft.

A distinctive trait of dry eye syndrome is the hyperosmolarity of the lacrimal film, which can damage the ocular surface, causing inflammation. Typically, the physiological osmolarity of the lacrimal film is less than 300 mOsm/L, while in subjects suffering from dry eye or keratoconjunctivitis sicca it reaches 360 mOsm/L. The hyperosmolarity of the lacrimal film triggers a cascade of signaling events with the release of inflammatory mediators, typically tumor necrosis factor, interleukin 1 and 6, which together cause damage to the ocular surface that can further reduce the stability of the lacrimal film, triggering a vicious cycle with chronicization of the disease.

In addition to hyperosmolarity, this pathological cycle can be triggered by other factors such as inflammation of the ocular surface, for example, caused by eye allergies, cellular toxicity of preservatives in topical formulations and xerophthalmia.

The symptoms of dry eye syndrome vary, ranging from a burning sensation and feeling of pressure in the eyes to a sensation of sand or presence of a foreign body, which can be accompanied by acute and dull pain localized in some area of the eye, behind the eye or even around the eye socket. Epiphora, or intermittent lacrimation, is an often counterintuitive symptom. This is a dryness that causes pain or irritation and results in excessive intermittent lacrimation. Additional symptoms include redness, often worsened by the rebound effect of the vasoconstrictors found in many over-the-counter eye drops, blurred vision, fluctuating vision, reading difficulty, feeling of heavy eyelids, difficulty in opening the eyes, tired eyes, excessive blinking, contraction of the eyelids. Moreover, eye dryness, together with irritation, can make contact lenses uncomfortable or even impossible to wear.

Moreover, as shown by Belmonte C. et al. in Ocul Surf; 2017, inflammation causes sensitization of the corneal nerve endings, with a consequent sensation of dryness and pain and, in the long term, changes in their excitability, overexpression and change in the activation threshold of ion channels, voltage-gated ion channels, and receptors, which can result in allodynia and hyperalgesia.

Treatment of dry eye syndrome generally involves a gradual approach which, depending on the severity of the symptoms, provides for education about the pathological condition, modification of environmental conditions, elimination of topical irritants and better eyelid hygiene.

The first line of therapeutic treatment provides for the administration of liquid preparations for ophthalmic use, tear substitutes and topical ocular lubricants. This topical treatment is fundamental in the chronic management of dry eye disease, since it requires compliance with a continuous, often multifactorial treatment. The results of this treatment are only appreciable over time, as documented by Lee BS et al. in Clin Ophthalmol., 2020.

However, the discomfort, local inflammation and pain at the instillation site, frequently reported in clinical practice, contribute to the lack of or improper compliance with therapy, with administration "as needed", which in turn perpetuates the discomfort of instilling eye drops and tear substitutes, generating a vicious circle in the management of this condition that requires chronic treatment. This becomes particularly important in subjects who must undergo polytherapeutic regimens with multiple instillations, in conditions of alteration of the ocular surface, for example following surgery or trauma, or use of topical products in formulations with preservatives that can alter the corneal epithelium or boost local inflammation.

Moreover, the state of inflammation causes sensitization of the corneal nerve endings, with a consequent sensation of dryness and pain and, in the long term, changes in their excitability, overexpression and modification of the activation threshold of ion channels, voltage-gated ion channels, and receptors, which can result in allodynia and hyperalgesia (Belmonte C. et al. Ocul Surf 2017).

When these problems become particularly serious, it becomes necessary to resort to a second line of treatment that provides for reversible punctal occlusion, the application of a night ointment or humidified goggles, heating the meibomian glands with suitable devices, intense pulsed light therapy, and in the most serious cases the administration of topical anti-inflammatory drugs such as corticosteroids, cyclosporine, lifitegrast and/or oral antibiotics, typically macrolides or tetracyclines. In the most severe cases that do not respond to the first line of treatment, ocular instillation of serum-based eye drops, oral or topical secretagogues, therapeutic contact lenses, amniotic membrane grafts, surgical punctal occlusion and tarsorrhaphy are used.

However, these treatments are not devoid of serious disadvantages in use, such as allergic reactions, exacerbation of existing inflammation, photophobia and adverse reactions.

Therefore, there is currently a need to have tear substitutes and moisturizing eye drops that are formulated in such a way as to reduce the number of cases of voluntary interruption of treatment or incomplete or irregular instillation.

An object of the present invention is to provide an ophthalmic preparation that is suitable for chronic treatment of dry eye disease with increased compliance to therapy with respect to the eye drops or tear substitutes in use.

Another object is to provide a tear substitute or eye drop for the treatment of dry eye that combines effective moisturizing action with reduced discomfort or pain during and after ocular instillation.

Another object is to provide an ophthalmic preparation for the treatment of dry eye or keratoconjunctivitis sicca whose use is substantially free of side effects that can be one of the causes of failure or improper compliance with chronic therapeutic treatment.

### SUMMARY

In accordance with certain aspects of the invention, it has been found that by formulating a selected active ingredient suitable for the treatment of peripheral and central neuropathic pain in combination with hyaluronic acid, preferably in specific concentrations, a synergistic anti-inflammatory effect is obtained which allows effective treatment of inflammatory disorders of the eye surface, specifically dry eye and keratoconjunctivitis sicca (KCS). The latter is a disease of the anterior segment of the eye which, like dry eye, features reduced production or excessive evaporation of tears. The most commonly presumed cause of KCS is immune-mediated destruction of the lacrimal gland, but other etiologies are also possible including neurogenic, infectious, congenital, secondary to systemic immunological disorders, induced by toxins or drugs, and iatrogenic. The effects of KCS include conjunctivitis and keratitis, which can lead to corneal changes that can also compromise visual function with chronicity. This pathology and its clinical course are broadly similar to those of dry eye.

Surprisingly, the combination composition described herein also showed synergistic activity in reducing the inflammatory mediators produced by corneal cells.

According to a general aspect, the present invention therefore provides an ophthalmic composition for the use as defined in claim 1.

Advantageously, the ophthalmic composition comprises hyaluronic acid and an ingredient that is active in reducing peripheral and central neuropathic pain and inflammation dispersed in a physiologically acceptable buffered aqueous solution.

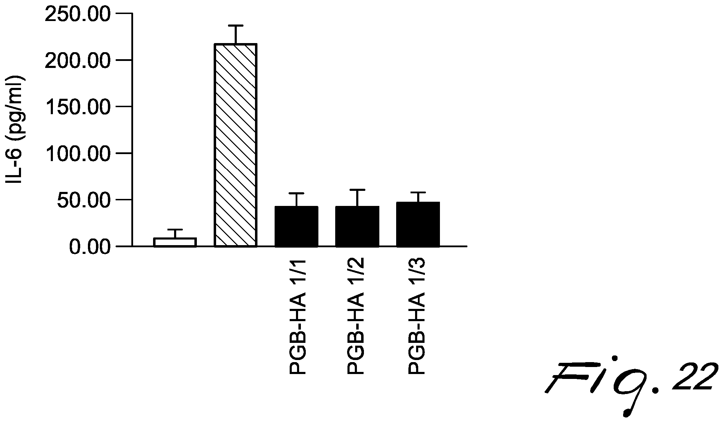

It has been observed that when the ophthalmic composition contains pregabalin and hyaluronic acid in a ratio of 1:10 to 3:1, preferably 1:1 to 1:3, an unexpected synergistic anti-inflammatory effect is observed, as shown by the data in the accompanying Figure 22. This synergistic effect is particularly appreciable in treating the symptoms associated with dry eye or keratoconjunctivitis sicca.

According to this embodiment, the ophthalmic composition described herein preferably contains pregabalin in an amount of 0.1% and hyaluronic acid in an amount of 0.3%. Preferably, the physiologically acceptable carrier is an aqueous solution that has characteristics that mimic those of natural tears, for example in terms of pH and osmolarity.

Preferably, the physiologically acceptable carrier comprises sterile water, preferably buffered. Preferably, the ophthalmic composition has the form of, or is formulated as, artificial tears or eye drops for topical use, typically for instillation into the outer structure of the eye.

For the purposes of the present invention, the ingredient that is active in the treatment of neuropathic pain through modulation of the release of excitatory neurotransmitters comprises pregabalin and salts or esters thereof.

In accordance with one aspect, the present invention relates to a water-based ophthalmic composition for use in the prevention and/or treatment of inflammatory disorders of the surface of the eye, comprising hyaluronic acid in a therapeutically effective amount in combination with pregabalin in a therapeutically effective amount and a physiologically acceptable vehicle.

Advantageously, instillation of the ophthalmic composition onto the surface of the eye reduces local inflammation and dry eye symptoms.

Within the scope of the present description, "compliance with therapy" is defined as the patient following the therapy for at least 80%; a compliance of less than 20% is defined as "resistance". Poor compliance with therapy reduces its effectiveness and can facilitate the onset of complications, prolongations or failure of the disease to remit.

Advantageously, the water-based ophthalmic composition described herein is formulated as eye drops suitable for instillation into the eye. Topical ophthalmic administration of the composition described herein reduces the discomfort and side effects typically encountered in the treatment of dry eye, increasing compliance with therapeutic treatment by the subject in need of treatment.

In particular, ophthalmic administration of the composition according to any of the embodiments described herein and combinations thereof, has an effective moisturizing action on the external structures of the eye, reducing the discomfort or pain that may occur during and after ocular instillation.

In accordance with some embodiments, the ophthalmic composition described herein may contain, in addition to the active ingredients dispersed in aqueous solution, one or more additional physiologically acceptable components, for example, a buffer substance or buffer system and/or one or more isotonic agents used in ophthalmic formulations, for example as described in detail below.

In accordance with some embodiments, the ophthalmic composition described herein may have a pH from slightly basic to neutral or slightly acidic, for example, a pH ranging from 6.4 to 7.6.

In accordance with some embodiments, the composition described herein may comprise one or more preservatives suitable for ophthalmic use.

Advantageously, the ophthalmic compositions described herein are pharmaceutical compositions containing active ingredients whose combination shows a surprising ability to act on ocular pathologies in which a contributing cause may be an alteration in the composition of the lacrimal film and/or insufficient production that determines or exacerbates diseases of the structure of the outer eye and in particular dry eye and/or keratoconjunctivitis sicca.

The topical use of the ophthalmic composition described herein, typically by ocular instillation into the conjunctival sac or on an external structure of the eye, allows to achieve a targeted ophthalmic therapy of dry eye and/or of the local inflammation that contributes to dry eye and inflammatory disorders of the surface of the eye, with high efficacy and a wide safety margin.

In accordance with certain embodiments, the ophthalmic composition described herein can be used in combination with other therapies commonly used in the treatment of one of the pathologies described herein.

In accordance with further aspects, the invention relates to an ophthalmic composition comprising hyaluronic acid optionally in salt form in combination with pregabalin in a therapeutically effective amount for topical use separately or sequentially with one or more ophthalmic medications to reduce the discomfort or pain associated with the multiple or continuous instillation of said ophthalmic medications and/or preservatives commonly used in formulations of ophthalmic preparations. In accordance with certain embodiments, said ophthalmic medications contain a non-steroidal anti-inflammatory drug (NSAID) or a steroidal drug, preferably a cortisone-based drug, an analog of topical prostaglandins, topical beta-blockers, direct-acting miotics, or topical cholinergic agonists.

A suitable non-steroidal anti-inflammatory drug is chosen from the group comprising ketorolac, diclofenac, indomethacin, nepafenac, flurbiprofen, bromfenac, piroxicam and their salts and/or mixtures.

A suitable steroidal anti-inflammatory drug is chosen from the group comprising dexamethasone, hydrocortisone, clobetasone and mixtures thereof.

In the context of the invention, the term "multiple instillation" means the repeated daily instillation of drops of an ophthalmic preparation, for example eye drops, on the ocular surface, conjunctiva or adnexa of the eye.

In accordance with certain embodiments, the ophthalmic composition comprising pregabalin and hyaluronic acid optionally in salt form according to any one of the embodiments described herein is for use in the treatment of an inflammatory disease of the ocular surface. In this therapeutic application, pregabalin and hyaluronic acid are preferably in a ratio in the range from 1:1 to 1:3 as described herein.

Advantageously, the composition described herein is a pharmaceutical composition or a medical device for ophthalmic use. Some aspects and effects of embodiments of the ophthalmic composition described herein become better apparent from the accompanying figures.

### DESCRIPTION OF FIGURES

Figures 1, 2, and 3 show IOP, Schirmer tear test, and corneal sensitivity in dogs suffering from corneal ulcers that received 0.3% hyaluronic acid, in particular:
   Figure 1 is a graphical representation of the mean variation in intraocular pressure in the animals (dogs) enrolled in the animal model clinical trials of Example 2;
Figure 2 is a graphical representation of the mean variation in tear production in the dogs enrolled in the animal model clinical trials of Example 2;
Figure 3 is a graphical representation of the mean variation in corneal sensitivity in AC dogs enrolled in the animal model clinical trials of Example 2. The values are expressed in mm of Chochet Bonnet filament length.
Figures 4, 5, and 6 show IOP, Schirmer tear test, and corneal sensitivity in dogs with corneal ulcers that received 0.01% pregabalin in 0.3% hyaluronic acid, in particular:

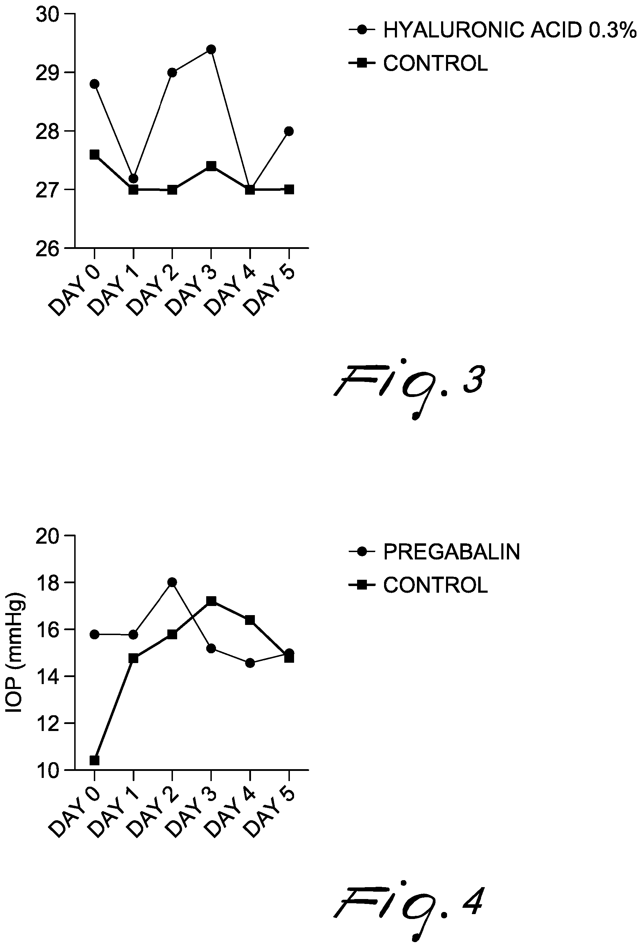

Figure 4 is a graphical representation of the mean variation in intraocular pressure in the dogs enrolled in the animal model clinical trials of Example 2;

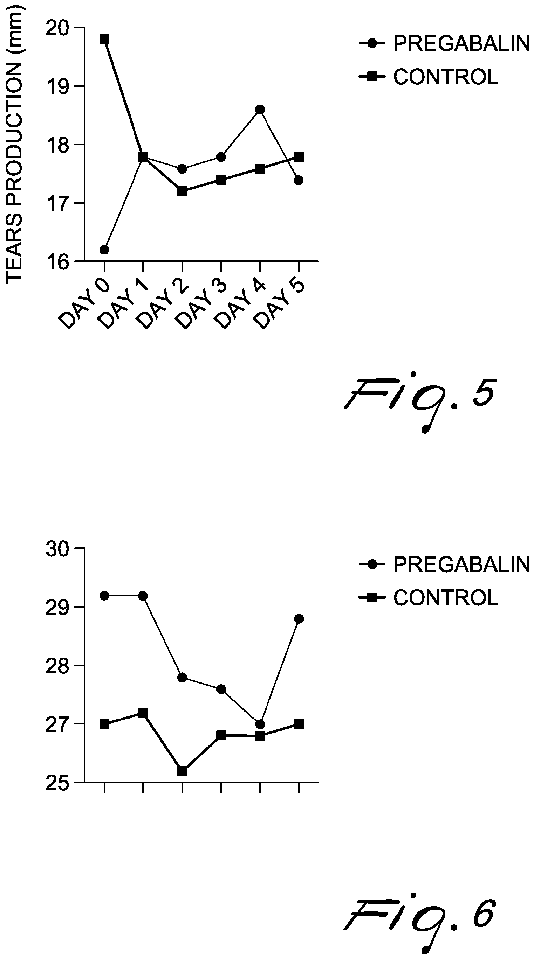

Figure 5 is a graphical representation of the mean variation in tear production in the AP dogs enrolled in the animal model clinical trials of Example 2.
Figure 6 is a graphical representation of the mean variation in corneal sensitivity in AP dogs enrolled in the animal model clinical trials of Example 2. The values are expressed in mm of Chochet Bonnet filament length.

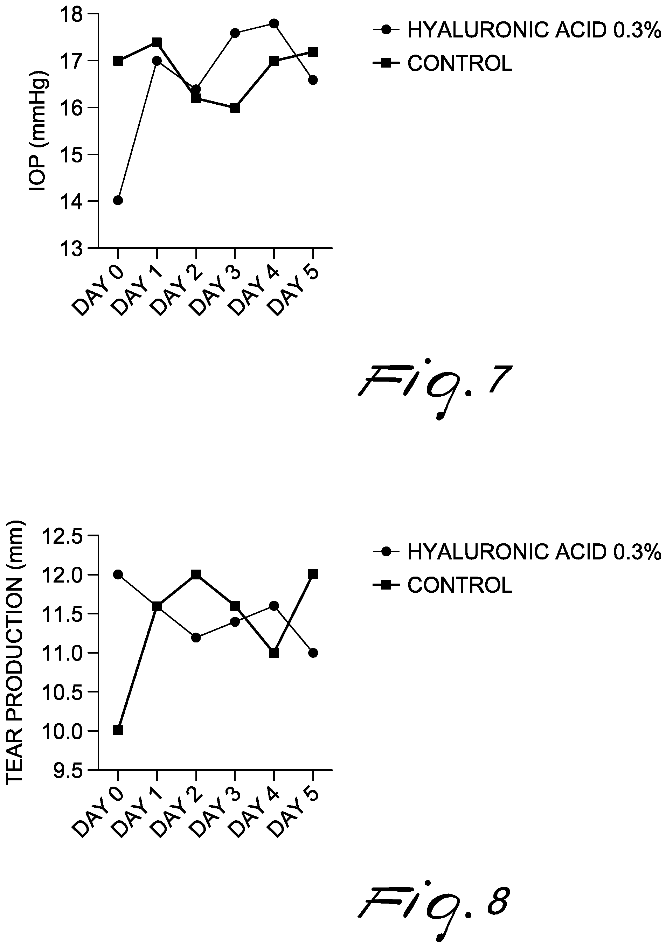

Figures 7, 8, and 9 plot IOP, Schirmer tear test, and corneal sensitivity in dogs suffering from keratoconjunctivitis sicca that received 0.3% hyaluronic acid. In particular:
   Figure 7 is a graphical representation of the mean variation in intraocular pressure in the CC dogs enrolled in the animal model clinical trials in Example 2.
Figure 8 is a graphical representation of the mean variation in tear production in the CC dogs enrolled in the animal model clinical trials of Example 2;

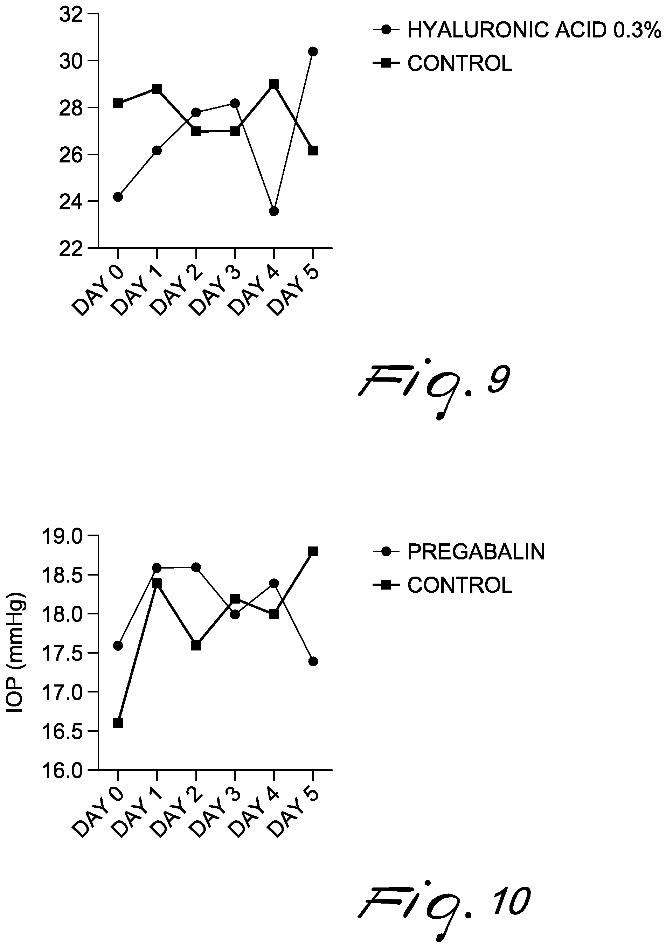

Figure 9 is a graphical representation of the mean variation in corneal sensitivity in the CC dogs enrolled in the animal model clinical trials in Example 2. The values are expressed in mm of Chochet Bonnet filament length.
Figures 10, 11, and 12 plot IOP, Schirmer tear test, and corneal sensitivity in dogs affected by keratoconjunctivitis sicca that received 0.01% pregabalin in 0.3% hyaluronic acid, in particular:
Figure 10 is a graphical representation of the mean variation in intraocular pressure in the CP dogs enrolled in the animal model clinical trials of Example 2;

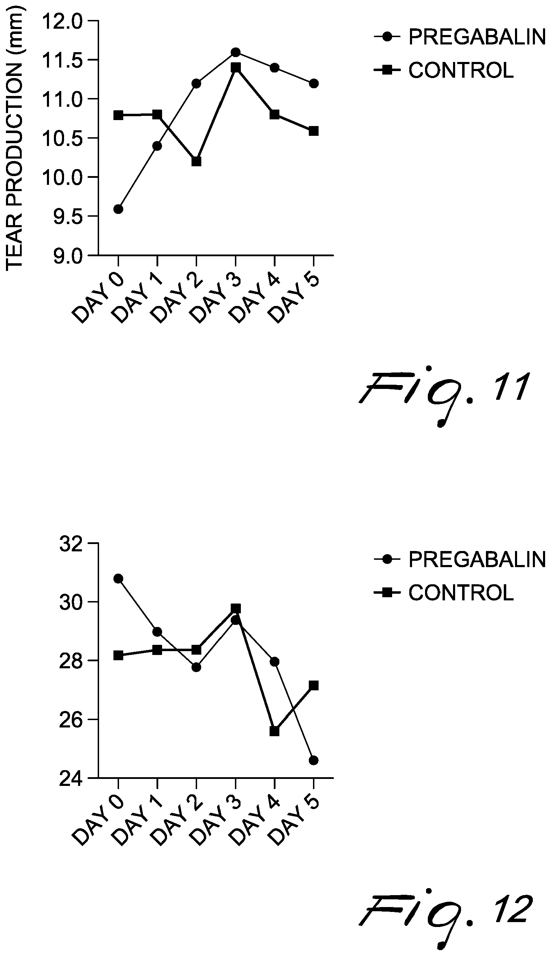

Figure 11 is a graphical representation of the mean variation in tear production in the CP dogs enrolled in the animal model clinical trials of Example 2.
Figure 12 is a graphical representation of the mean variation in corneal sensitivity in CP dogs enrolled in the animal model clinical trials of Example 2. The values are expressed in mm of Chochet Bonnet filament length.

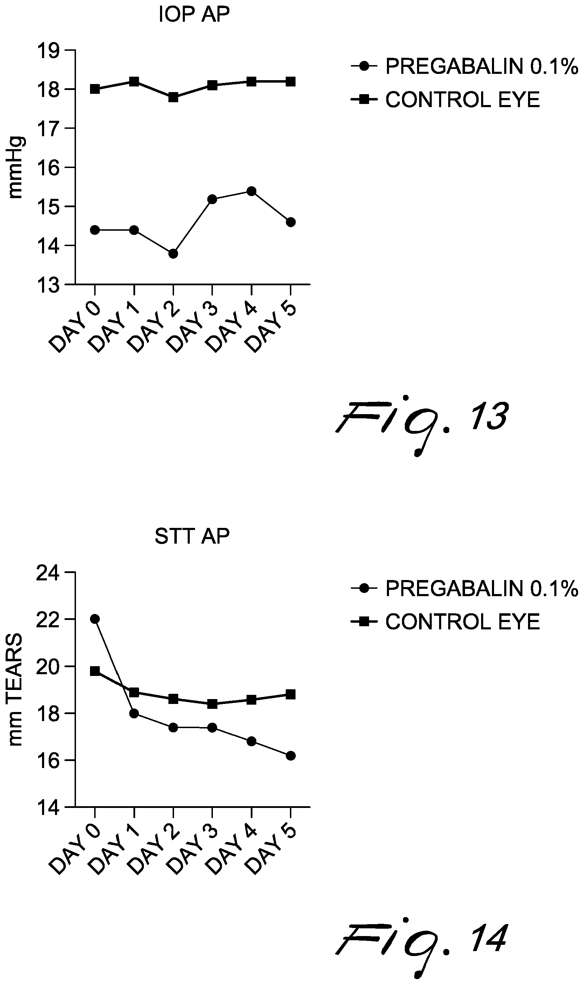

Figures 13, 14, and 15 plot IOP, Schirmer tear test, and corneal sensitivity in dogs with corneal ulcers that received 0.1% pregabalin in 0.3% hyaluronic acid, in particular:
Figure 13 is a graphical representation of the mean variation in intraocular pressure in AP dogs (0.1% pregabalin) enrolled in the animal model clinical trials of Example 2;
Figure 14 is a graphical representation of the mean variation in tear production in AP dogs (0.1% Pregabalin) enrolled in the animal model clinical trials of Example 2;

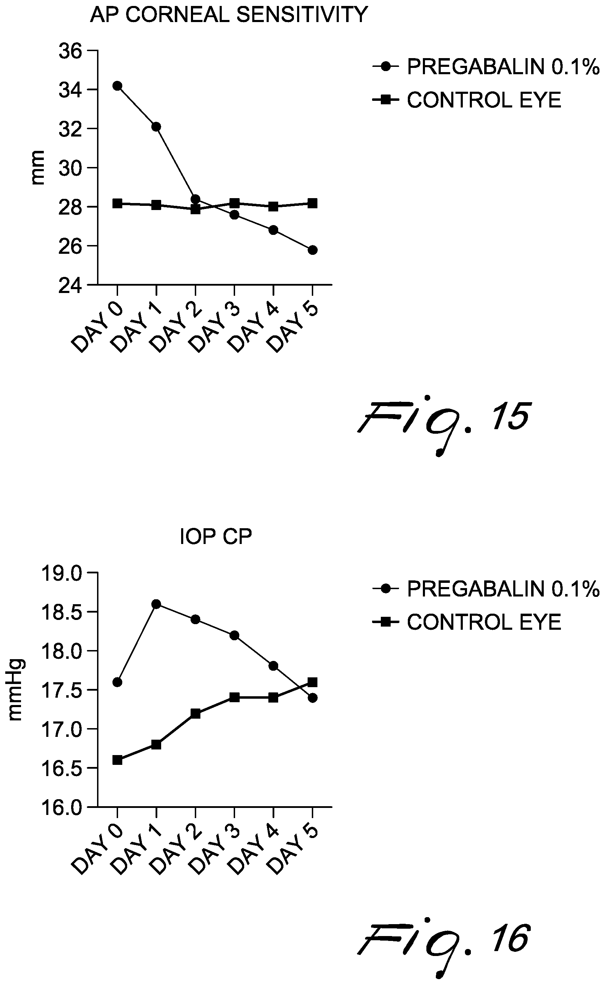

Figure 15 is a graphical representation of the mean variation in corneal sensitivity in AP dogs (0.1% pregabalin) enrolled in the animal model clinical trials of Example 2;
Figures 16, 17, and 18 plot IOP, Schirmer tear test, and corneal sensitivity for dogs suffering from keratoconjunctivitis sicca who received 0.1% pregabalin in 0.3% hyaluronic acid, in particular:
Figure 16 is a graphical representation of the mean variation in intraocular pressure in CP dogs (0.1% pregabalin) enrolled in the animal model clinical trials of Example 2;

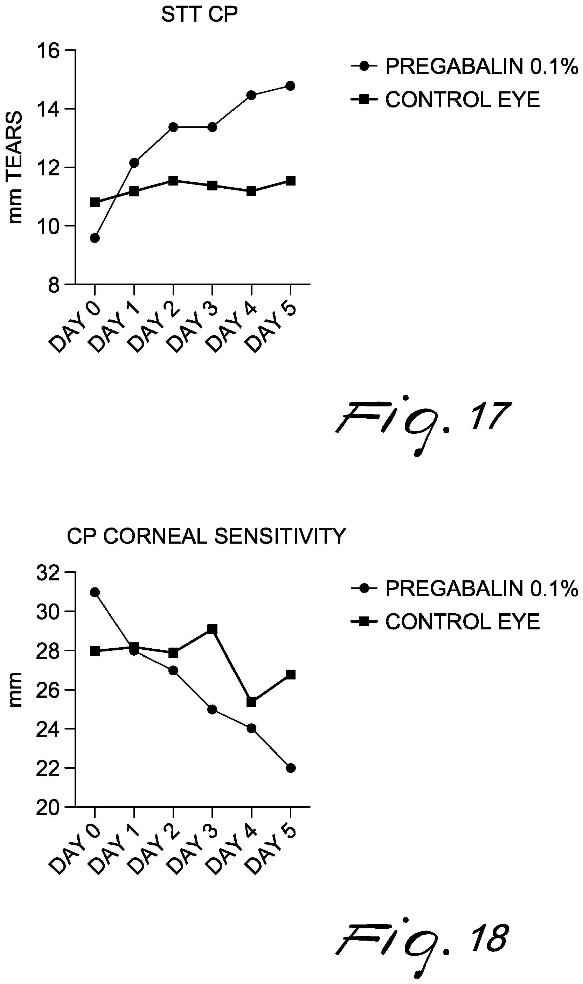

Figure 17 is a graphical representation of the mean variation in tear production in CP dogs (0.1% pregabalin) enrolled in the animal model clinical trials of Example 2;
Figure 18 is a graphical representation of the mean variation in corneal sensitivity in CP dogs (0.1% pregabalin) enrolled in the animal model clinical trials of Example 2;

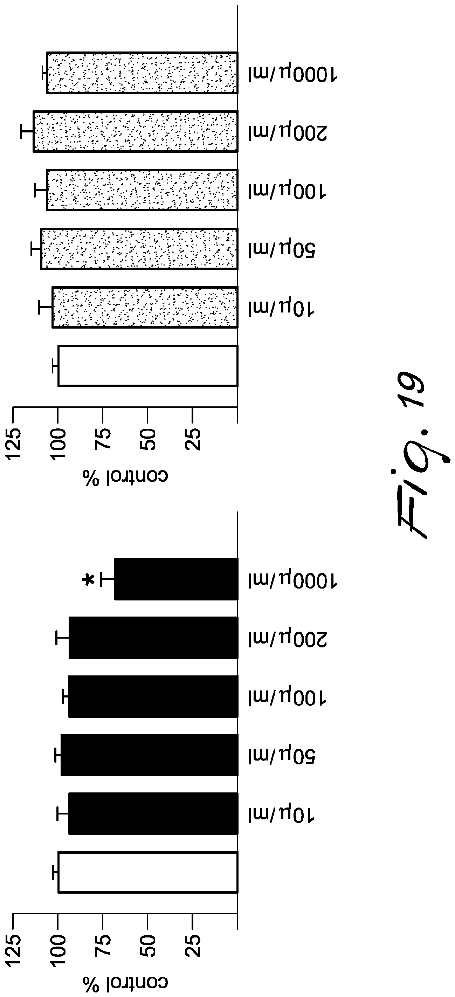

Figure 19 shows bar charts related to the MTT assay of Example 4 in which the results are expressed as a percentage of the control in duplicate cultures in human corneal epithelial cells. The bar charts show that the state of preservation of the HCECs cells in the cultures treated with PGB and HA, did not show toxicity after treatment at 10, 50, 100, 200 µg/ml of the tested substances;

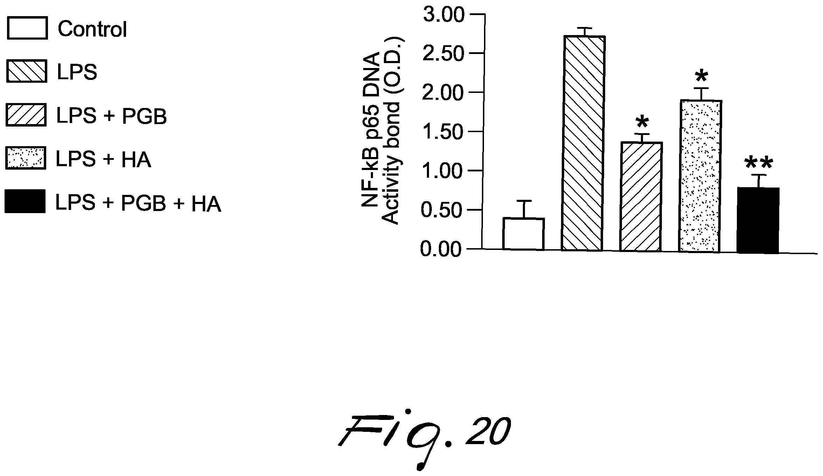

Figure 20 shows bar charts related to the control, the sample with lipopolysaccharide LPS, the LPS+PGB sample, the LPS+HA sample and the sample with LPS and the combination composition PGB+HA. The charts show how the combination of pregabalin (PGB) and hyaluronic acid (HA) [black chart] has a synergistic effect of reducing NF-kβ p65 binding activity in human macrophage cultures exposed to LPS, as described in Comparative Example 4;

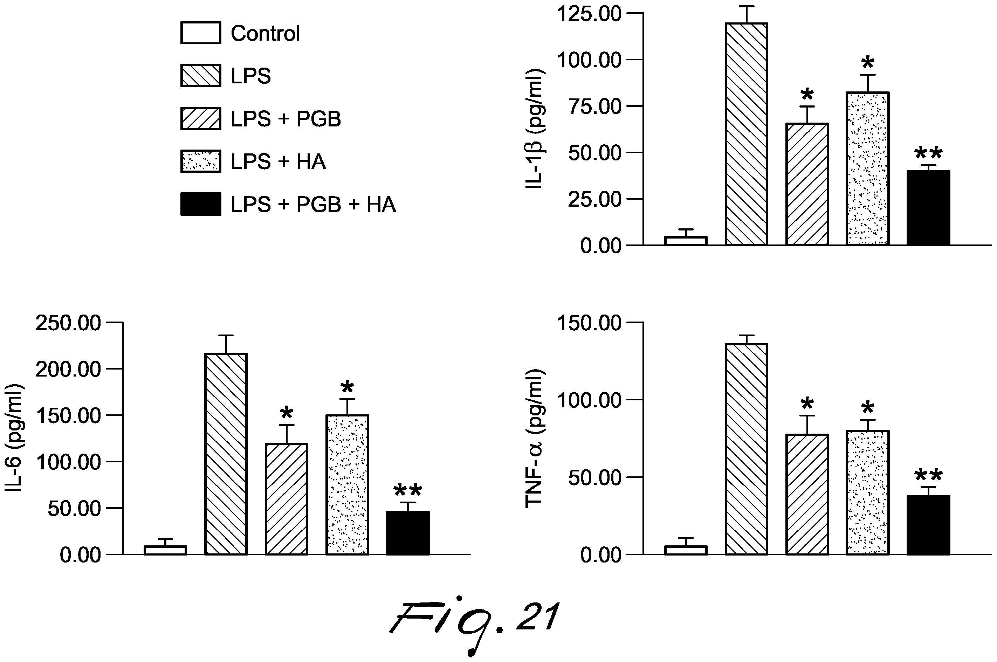

Figure 21 shows bar charts related to the control, the sample with lipopolysaccharide LPS, the sample with LPS+PGB, the sample with LPS+HA and the sample with LPS and the combination composition PGB+HA. The charts show the synergistic effect of the PGB + HA combination (black bar chart) in reducing the secretion of inflammatory cytokines in human corneal epithelial cell culture exposed to LPS, according to Example 4;
Figure 22 shows bar charts that highlight the synergistic anti-inflammatory effect on corneal cells tested with a combination of PGB and HA in a ratio from 1:1 to 1:3.

### DETAILED DESCRIPTION OF THE INVENTION

The present invention originates from the finding that instilling an ophthalmic composition containing hyaluronic acid and a selected ingredient, which is active in the modulation of nociceptive transmission, dispersed in a physiologically acceptable carrier in liquid form, reduces symptoms of dry eye or keratoconjunctivitis sicca as well as the pain and discomfort associated with ocular instillation and, unexpectedly, in a synergistic manner, the inflammation of the corneal cells that amplifies local sensitivity.

In accordance with one aspect, the invention relates to an ophthalmic composition for treating dry eye or keratoconjunctivitis sicca comprising a liquid carrier in which hyaluronic acid and an ingredient that is active in the treatment of neuropathic pain are dispersed, both present in a therapeutically effective amount.

An ophthalmic composition as defined in claim 1 therefore constitutes an object of the present invention.

Preferably, the ophthalmic composition described herein is in liquid form of a water-based solution or gel.

Further embodiments of the ophthalmic composition are defined in the appended dependent claims 2-9.

Another object of the invention is an ophthalmic composition comprising pregabalin and hyaluronic acid, optionally in salt form, and a physiologically acceptable vehicle, preferably in liquid or gel form, for medical uses in the ophthalmic field as formed by claims 10-15. In the context of these ophthalmological uses of the ophthalmic composition described herein, preferably pregabalin and hyaluronic acid are in a ratio in the range from 1:1 to 1:3.

One of the active ingredients contained in the ophthalmic composition described herein is hyaluronic acid, optionally in a salified form.

Hyaluronic acid is typically a glycosaminoglycan that provides for the repetition of disaccharide units formed by glucuronic acid joined by means of a β1→4 and β1→3 glycosidic bond with N-acetylglucosamine.

A suitable hyaluronic acid may be in a free non-cross-inked form or in a salified form with a physiologically acceptable salt, for example sodium or potassium hyaluronate. Hyaluronic acid is preferably in a free salified form such as sodium hyaluronate.

In accordance with certain embodiments, the ophthalmic composition described herein comprises hyaluronic acid, optionally in a linear or cross-inked salified form, in an amount from 0.001% to 3% by weight, preferably from 0.01% to 1%, more preferably from 0.1% to 0.6% by weight, for example 0.3%.

A suitable hyaluronic acid or salt thereof may have a medium-high molecular weight.

For example, a suitable hyaluronic acid or salt thereof may have an average molecular weight greater than 300,000 Da, preferably comprised between 1,000,000 and 6,000,000 Dalton from 1,500,000 to 3,500,000 Dalton. In certain embodiments, the ophthalmic composition comprises hyaluronic acid with at least two different molecular weights or cross-inked hyaluronic acid with a suitable physiologically acceptable cross-linking agent.

Typically, the molecular weights of hyaluronic acid or salts thereof, as described herein, are expressed as weight average molecular weight (MW), expressed in Daltons. The average molecular weight of the hyaluronic acid described herein can be determined using standard methods commonly used in the pharmaceutical formulation sector, for example as described by Ueno et al., 1988, Chem Pharm Bull. 36, 4971-4975; Wyatt 1993, Anal Chim Acta 272: 1-40; Watt Technologies 1999 "Light scattering University Dawn Course Manual and 'Dawn Eos Manual'" Wyatt Technology Corp. Santa Barbara CA (USA).

Hyaluronic acid, being rich in hydroxyl groups that attract and retain water molecules, thickens and stabilizes the lacrimal film. Moreover, hyaluronic acid reduces the effects of mechanical trauma on the ocular surface by means of lubrication and by contributing to re-epithelialization. Moreover, hyaluronic acid reduces the rate of tear evaporation from the ocular surface, preventing and reducing the symptoms of dry eye and/or dry keratoconjunctivitis.

A further component contained in the ophthalmic composition described herein is an ingredient with an effect on the modulation of nerve transmission and which is indicated, among other things, in the treatment of peripheral and central neuropathic pain, preferably pregabalin. These molecules are structural analogues of the neurotransmitter γ-aminobutyric acid (GABA) without pharmacological activity on GABA receptors.

Pregabalin is an analogue [(S)-3-(aminomethyl)-5-methylhexanoic acid] of gamma-aminobutyric acid that binds to the accessory subunit (α2-δ protein) of the voltage-dependent calcium channels in the nervous system.

Advantageously, pregabalin is useful in modulating painful nerve transmission. The pharmacological effect of pregabalin is mainly due to its action on receptor targets present in the central nervous system; however, the extensive corneal innervation, the ubiquitous expression of the α2δ subunit, and of the voltage-dependent calcium channels in the peripheral nerve endings, including the corneal ones, provides a transient effect on nociceptive transmission when applied topically, for example by ocular instillation. It has now been observed that this effect reduces the sensation of discomfort generated by corneal stimulation when instilling ophthalmic preparations, for example eye drops.

In particular, the gabapentinoid described here has an analgesic effect through the antagonistic activity of voltage-dependent Ca2+ channels, with the type I α2-δ subunit of voltage-dependent Ca2+ channels in the central nervous system as the main antagonistic target. The gabapentinoid bond inhibits cellular calcium influx and attenuates neurotransmission, for example by reducing the release of substance P, CGRP, glutamate or noradrenaline. Despite the similar mechanism of action, pregabalin has pharmacokinetic advantages over gabapentin, together with a greater binding affinity for α2-δ and a more powerful analgesic action.

The local administration of gabapentinoids provides significant pain relief and has been associated with a reduction in allodynia, hyperalgesia or dysesthesia, both thermal and mechanical, induced by experimental models, without central nervous system-mediated side effects such as impairment of locomotor function.

The presence of pregabalin in the ophthalmic composition described herein allows a reduction in the discomfort associated with the frequent administration of ophthalmic preparations in the population of individuals suffering from inflammatory eye diseases, for example dry eye or keratoconjunctivitis sicca, thus improving compliance with therapeutic treatment.

It has been observed that the formulation of pregabalin with hyaluronic acid in an ophthalmic preparation reduces the pain and the sensation of discomfort generated by corneal stress, also consequent to excessive local inflammatory response, which accompanies instillation, in particular repeated daily instillation, for example from 2 to 10 times a day. This effect is used as the preferred method for preventing or reducing the pain and discomfort associated with the repeated instillation of eye drops or artificial tears to treat dry eye or keratoconjunctivitis sicca and/or an inflammatory eye disease, of local or systemic origin.

Consequently, an ophthalmic composition comprising hyaluronic acid and pregabalin dispersed in a water-based liquid vehicle, typically an aqueous solution, finds application in the treatment of dry eye and/or keratoconjunctivitis sicca and/or other conditions requiring lubrication of the ocular surface, including surgical outcomes, and in preventing or reducing the discomfort or pain associated with for example continuous or repeated instillation of the ophthalmic composition into the eye. Advantageously, the use of the composition described herein improves compliance with a chronic treatment regimen for dry eye and/or dry keratoconjunctivitis.

In accordance with certain embodiments, the composition contains pregabalin in an amount from 0.005 to 3% by weight, from 0.01 to 1% from 0.05 to 0.2% by weight with respect to the total weight of the composition.

Moreover, it has been observed that a composition comprising a gabapentinoid, preferably pregabalin, and hyaluronic acid and a liquid carrier according to any of the embodiments described herein, increases compliance with a chronic ophthalmic treatment regimen of the eye with ophthalmic medications adapted, for example, to treat dry eye or keratoconjunctivitis sicca.

This effect and the other effects mentioned herein are achieved more markedly by instilling an ophthalmic composition containing pregabalin in a concentration of 0.05% to 0.2% and hyaluronic acid in a concentration of 0.1% to 0.6% by weight on the ocular surface.

In accordance with certain embodiments, the ophthalmic composition described herein has a pH in the range from 6.4 to 7.6 using a suitable buffer or buffer system and b) the possible presence in the solution of divalent calcium and magnesium salts such as magnesium chloride and calcium chloride.

The Applicant has also observed that effective regulation of the pH within the desired range can be obtained by using a buffer or buffering system, for example, selected from sodium hydrogen phosphate, in particular monohydrate, disodium phosphate, in particular dodecahydrate, and mixtures thereof.

In accordance with some embodiments, the ophthalmic composition described herein comprises a further buffer, for example sodium citrate, boric acid/borate.

In accordance with some embodiments, the composition described herein may comprise an isotonic agent, for example selected from magnesium chloride, calcium chloride, sodium chloride and mixtures thereof.

In accordance with some embodiments, the composition of the invention may comprise an additional isotonic agent such as glycerol.

Advantageously, the excipient or co-formulating components buffer and isotonic agent may be present in an amount such as to obtain a pH between 6.4 and 7.6.

In accordance with some aspects, the ophthalmic composition described herein can be in a single-dose or multi-dose dosage form.

Advantageously, the ophthalmic composition described herein can be isotonic with the lacrimal fluid and can preferably have a value comprised between 260-320 mOsm/L, more preferably comprised between 275 and 300 mOsm/L.

Preferably, the composition according to the invention is ophthalmic eye drops or gel. For the purpose of administration, said compositions can be provided as eye drops or in liquid or gel form.

The compositions described here, formulated as multi-dose, may also contain preservatives with antimicrobial activity. Typical examples include parabens, quaternary ammonium salts, NIG/EDTA, polyhexamethylene biguanide (PHMB) and mixtures thereof.

Preferably, the ophthalmic composition is free of preservatives.

Advantageously, the solvent used in the composition described herein is water or an aqueous solution of one or more liquids compatible with ophthalmic use.

In the present context, the terms dry eye and dry eye syndrome have the same meaning and as such are interchangeable. As used here, the term combination means that the components or active ingredients of the composition described herein are both present, for example in the form of a mixture, without any chemical bond or other chemical-physical interaction forming between them.

The following examples further illustrate some aspects of the invention.

### EXAMPLE 1

A tear substitute or eye drops adapted in the treatment of dry eyes comprises 0.2% sodium hyaluronate; food grade monobasic potassium phosphate 0.06%; disodium hydrogen phosphate 0.639; hydrochloric acid 0.1N ~2.495; sodium chloride 0.574; purified water Q.S. to 100.

### EXAMPLE 2

An ophthalmic composition, for example in the form of single-dose eye drops, for the treatment of dry eye syndrome and/or keratoconjunctivitis sicca having the following formulation:

| COMPONENT | % | FUNCTION |
|---|---|---|
| SODIUM HYALURONATE (of which HYALURONIC ACID) | 0.307 (0.300) | Moisturizing / emollient / lubricant |
| PREGABALIN PH. EUR. | 0.100 | Reduce discomfort during instillation |
| FOOD GRADE MONOBASIC POTASSIUM PHOSPHATE | 0.060 | Buffering agent |
| DISODIUM HYDROGEN PHOSPHATE | 0.639 | Buffering agent |
| HYDROCHLORIC ACID 0.1N (for pH adjustment) | ~2.495 | pH correcting agent |
| SODIUM CHLORIDE PH.EUR - USP | 0.574 | Osmolarity correcting agent |
| Purified water | q.s. to 100 | Solvent |

### EXAMPLE 3

Evaluation of pregabalin for topical/ophthalmic application in a spontaneous animal model.

Specifically, the therapeutic effect of an ophthalmic preparation based on hyaluronic acid and pregabalin in the treatment of corneal diseases such as dry eye/dry keratoconjunctivitis was tested on an animal model.

### Purpose of the investigation

An evaluation was carried out on the effect of topical pregabalin mixed with hyaluronic acid as eye drops in reducing ocular pain in veterinary patients suffering from chronic and acute painful corneal disease. To test the effect of these molecules, corneal ulceration and keratoconjunctivitis sicca, which is completely comparable to dry eye in humans, were used as spontaneous models.

### Materials and methods

A total of 20 dogs were enrolled, ten with corneal ulcers (acute spontaneous model) and ten with keratoconjunctivitis sicca (KCS, chronic spontaneous model).

At the time of enrollment, all dogs underwent a specialized ophthalmological examination comprising slit lamp evaluation, Schirmer tear test (STT), tonometry (IOP; measured daily with a portable tonometer; Tonopen Vet, Reichert, Inc., Depew, NY USA), corneal esthesiometry, evaluation of the ocular fundus by direct and indirect ophthalmoscopy.

More specifically, corneal sensitivity was measured with the Cochet-Bonnet esthesiometer (Luneau Ophtalmologie, Chartres Cedex, France) using a previously described protocol.

Data collection began approximately two weeks after the screening examination and to eliminate problems related to different mechanical properties (for example stiffness and flexibility) between older and newly manufactured filaments, a new filament was obtained and used during the study. Briefly, starting with a filament length of 40 mm, the instrument was advanced at right angles towards the central cornea until a slight deflection of the filament was noted after corneal contact. If the blink reflex was not observed in at least three out of five attempts, the filament length was reduced in 2-mm increments and the procedure was repeated. The filament length is inversely proportional to corneal sensitivity (that is to say, a more sensitive cornea will elicit a blink response with a longer filament length) and corneal sensitivity is expressed as the shortest filament length in mm that induced a blink response on three out of five measurements. In an attempt to minimize environmental variability (for example, lighting conditions, temperature, humidity) and procedural variability on corneal esthesiometry, all measurements were performed in the same examination room by a single experimenter. Minimal and gentle manual restraint was used for all study subjects.

As regards the acute spontaneous model, the ten dogs suffering from corneal ulcer (A) were divided into two groups: control (AC) and Pregabalin (AP).

The control dogs (AC) received daily administration of eye drops containing 0.3% hyaluronic acid for five days, while the pregabalin dogs (AP) received daily administration of eye drops containing 0.01% pregabalin in 0.3% hyaluronic acid. In each dog only one eye was treated, using the contralateral one as a control.

As regards the chronic spontaneous model, the ten dogs suffering from keratoconjunctivitis sicca (C) were divided into two groups: control (CC) and Pregabalin (CP).

The control dogs (CC) received daily administration of eye drops containing 0.3% hyaluronic acid for five days, while the pregabalin dogs (CP) received daily administration of eye drops containing 0.01% pregabalin in 0.3% hyaluronic acid. In each dog only one eye was treated, using the contralateral eye as a control.

All dogs underwent STT, tonometry and corneal sensitivity evaluation on a daily basis for five consecutive days.

Ten more dogs (4 in the AP group and 6 in the CP group) were enrolled for the evaluation of eye drops with 0.1% pregabalin (results in Appendix 1).

### Results

### Corneal ulceration

The results observed are shown in the charts in the accompanying figures.

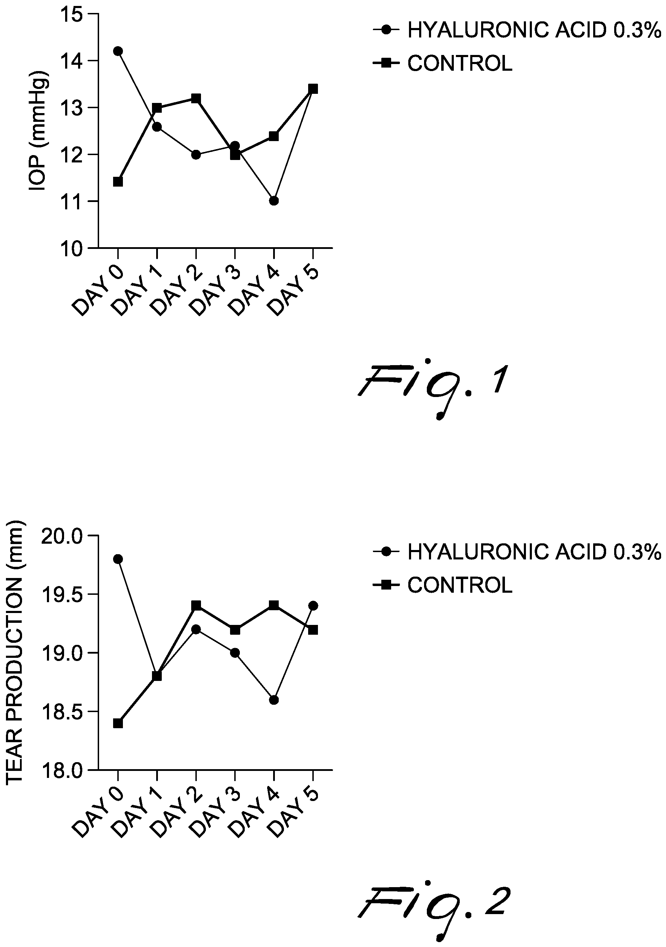

In particular, Figures 1, 2 and 3 show intraocular pressure, Schirmer's tear test and corneal sensitivity respectively for the dogs with a corneal ulcer treated with 0.3% hyaluronic acid.

Figures 4, 5 and 6 show intraocular pressure, the Schirmer tear test and corneal sensitivity for dogs with corneal ulcers treated with pregabalin in 0.3% hyaluronic acid, respectively.

The data obtained was analyzed using ANOVA, which did not show any statistical differences between the groups. It is likely that the trend recorded for corneal ulcer is correlated to the progression/evolution of the disease itself rather than to the therapeutic interventions. Keratoconjunctivitis sicca

Figures 7, 8 and 9 show intraocular pressure, Schirmer's tear test and corneal sensitivity respectively for dogs suffering from KCS treated with 0.3% hyaluronic acid.

Figures 10, 11 and 12 show intraocular pressure, the Schirmer tear test and corneal sensitivity, respectively, for dogs suffering from KCS treated with pregabalin in 0.3% hyaluronic acid.

The data obtained were analyzed by means of ANOVA, a different statistical tear production was evidenced for the dogs treated with 0.3% hyaluronic acid on its own; no other statistically significant differences were found.

The marked tendency for improvement in corneal sensitivity recorded in dogs treated with 0.01% pregabalin in 0.3% hyaluronic acid should be noted. In detail, on Day 0 the length of the Chochet Bonnet filament capable of causing these dogs to blink (threshold limit for corneal sensitivity) was 31 mm (pressure of 1.4 g/mm²) while on Day 5 it rose to 24.5 mm (pressure of 1.9 g/mm²).

A comparison of the data obtained by administering 0.1% pregabalin and 0.01% pregabalin in the AP and CP dogs is shown in tables 1 and 2 respectively.

It is possible to note that 0.1% pregabalin was more able than 0.01% pregabalin to decrease the discomfort of the dogs (understood as a decrease in tear production, STT and restoration of normal corneal sensitivity) during corneal ulceration, without having any effect on intraocular pressure. Similarly, 0.1% pregabalin was more able than 0.01% pregabalin to improve tear production in dogs suffering from KCS (+4.8 mm vs +1.6 mm STT) and to restore normal corneal sensitivity (from 31 mm to 22 mm of Chochet Bonnet filament length for 0.1% pregabalin vs from 30.8 mm to 24.6 mm of Chochet Bonnet filament length for 0.01% pregabalin), with no effect on intraocular pressure.

**Table 1 below shows the STT, IOP and corneal sensitivity data for AP dogs treated with 0.1% pregabalin and 0.01% pregabalin. For each parameter, the percentage variation on Day 5 compared to Day 0 is reported.**

| *STT (mm)* | | | *IOP (mmHg)* | | | *CORNEAL SENSITIVITY (mm)* | | |
|---|---|---|---|---|---|---|---|---|
| | **0.1% pregabalin** | **0.01% pregabalin** | | **0.1% pregabalin** | **0.01% pregabalin** | | **0.1% pregabalin** | **0.01% pregabalin** |
| Day 0 | 22 | 16.2 | Day 0 | 14.4 | 15.8 | Day 0 | 34.2 | 29,2 |
| day 1 | 18 | 17.8 | day 1 | 14.4 | 15.8 | day 1 | 32.1 | 29,2 |
| day 2 | 17.4 | 17.6 | day 2 | 13.8 | 18 | day 2 | 28.4 | 27,8 |
| day 3 | 17.4 | 17.8 | day 3 | 15.2 | 15.2 | day 3 | 27.6 | 27,6 |
| day 4 | 16.8 | 18.6 | day 4 | 15.4 | 14.6 | day 4 | 26.8 | 27 |
| Day 5 | 16.2 | 17.4 | Day 5 | 14.6 | 15 | Day 5 | 25.8 | 28,8 |
| | **74%** | **107%** | | **101%** | **95%** | | **75%** | **99,00%** |
| | **Variation Day 5 / Day 0** | | | **Variation Day 5 / Day 0** | | | **Variation Day 5** / **Day 0** | |

**Table 2 below shows the STT, IOP and corneal sensitivity data for CP dogs treated with 0.1% pregabalin and 0.01% pregabalin. For each parameter, the percentage change on Day 5 compared to Day 0 is shown.**

| *STT (mm)* | | | *IOP (mmHg)* | | | *CORNEAL SENSITIVITY (mm)* | | |
|---|---|---|---|---|---|---|---|---|
| | **0.1% pregabalin** | **0.01% pregabalin** | | **0.1% pregabalin** | **0.01% pregabalin** | | **0.1% pregabalin** | **0.01% pregabalin** |
| Day 0 | 9.6 | 9.6 | Day 0 | 17.6 | 17.6 | Day 0 | 31 | 30.8 |
| day 1 | 12.2 | 10.4 | day 1 | 18.6 | 18.6 | day 1 | 28 | 29 |
| day 2 | 13.4 | 11.2 | day 2 | 18.4 | 18.6 | day 2 | 27 | 27.8 |
| day 3 | 13.4 | 11.6 | day 3 | 18.2 | 18 | day 3 | 25 | 29.4 |
| day 4 | 14.5 | 11.4 | day 4 | 17.8 | 18.4 | day 4 | 24 | 28 |
| Day 5 | 14.8 | 11.2 | Day 5 | 17.4 | 17.4 | Day 5 | 22 | 24.6 |
| | **154%** | **117%** | | **99%** | **99%** | | **71%** | **80,00%** |
| | **Variation Day 5 / Day 0** | | | **Variation Day 5 / Day 0** | | | **Variation Day 5 / Day 0** | |

### Discussion

In the present test, pregabalin was able to improve corneal sensitivity in dogs suffering from KCS, restoring physiological sensitivity. It can be hypothesized that this occurred through modulation of pain perception.

The results obtained in the evaluation presented above lay the foundation for a new therapeutic approach of modulation of corneal pain perception, with the aim of providing comfort to patients and offering local and transient analgesia without delaying epithelialization or negatively affecting tear production.

The specific mechanism by which pregabalin affects corneal wound healing, corneal sensitivity and tear production is currently unknown in dogs.

In conclusion, 0.1% pregabalin appears to be more effective than 0.01% in decreasing discomfort, understood as restoration of physiological lacrimation and corneal sensitivity, of dogs suffering from corneal diseases such as ulceration, KCS and dry eye.

### Further evidence of therapeutic activity in the animal model

The accompanying Figures 13, 14 and 15 show intraocular pressure, the Schirmer tear test and corneal sensitivity for dogs with corneal ulcers treated with 0.1% pregabalin in 0.3% hyaluronic acid, respectively.

A marked tendency is noted towards improvement in STT and corneal sensitivity recorded in dogs treated with 0.1% pregabalin in 0.3% hyaluronic acid.

Figures 16, 17 and 18 show intraocular pressure, the Schirmer tear test and corneal sensitivity for dogs suffering from KCS that received 0.1% pregabalin in 0.3% hyaluronic acid respectively.

A marked tendency is noted toward improvement in STT and corneal sensitivity recorded in dogs treated with 0.1% pregabalin in 0.3% hyaluronic acid.

### EXAMPLE 4

### Purpose of the experimental tests

An in vitro experimental inflammation model was used to verify the synergistic inhibitory effect of the combination of pregabalin (PGB) and hyaluronic acid (HA) on the expression of pro-inflammatory cytokines involved in the etiopathogenesis of dry eye.

### Materials and methods

The primary culture of HCEC (cat. PCS-700-010) was purchased from the American Type Culture Collection (ATCC), Manassas, VA, USA.

The cells were resuspended in basal medium for corneal epithelial cells (devoid of serum and calcium) and supplemented with a growth kit provided by ATCC.

### Cell viability

The cells were seeded at a density of 8 × 10³ cells/well in a 96-well plate and cultured overnight. The cells were then treated with PGB or HA at different concentrations for 24 hours or with empty medium (control). After gently washing them with PBS, the cells were incubated with 0.5 mg/ml MTT solution in the dark for 4 hours. After removing the supernatant, DMSO was added to solubilize the formazan. Absorbance at 570 nm was measured with an ELISA reader (Multiskan MK3) for cell viability.

As shown in Figure 19, the MTT assay shows that HCEC cells were well preserved in both PGB- and HA-treated cultures, with no evidence of toxicity after treatment at 10, 50, 100, 200 µg/ml. At 1000 µg/ml PGB induces a certain reduction in cell viability, while HA has no evident toxicity even at higher dosages. All data are represented as mean ± SEM of three independent experiments.

### Test

### Preamble

It is known from scientific literature that the NF-kβ pathway plays a central role in the etiology of chronic inflammatory diseases. The family of NF-kβ transcriptional factors plays a critical role in regulating the expression of a wide variety of genes, in particular in the inflammatory process, including cytokines such as IL-1β, IL-6 and TNF-α.

### Experimental tests

To evaluate the ability of the synergistic composition of the invention to inhibit NF-kβ activity in corneal cells, the NF-kβ p65 DNA Binging activity test was used with the ELISA kit of the TransAM p65 transcription factor (Active Motif, Carlsbad, CA).

It has been demonstrated that lipopolysaccharide (LPS) accumulated in gram-negative bacteria, effectively induces inflammation in the corneas of corneal lesion models. HCEC cells were stimulated with 500 ng/ml LPS in the presence and absence of PGB 100 µg/ml, HA 100 µg/ml or a combination of PGB+HA for 12 hours, as described here.

Subsequently, the DNA binding activity of NF-kβ p65 was determined. 12 hours after stimulation with LPS markedly promoted the DNA-binding activity of NF-kβ p65 and the expression of the pro-inflammatory cytokines IL-1β, IL-6 and TNF-α. However, a lower DNA-binding activity was observed in LPS-stimulated macrophages in the presence of PGB and/or HA, as shown in Figure 20.

As can be seen in Figure 20, the combination of PGB+HA strongly reduced the DNA-binding activity of NF-kβ p65, demonstrating a synergistic effect obtained by the combination of the two compounds. Accordingly, similar effects were observed in terms of cytokine expression (Figure 22).

IL-1b, IL-6 and TNF-a were detected with ELISA kits purchased from Abcam, Cambridge (MA) USA. Data are expressed as mean ± SEM, n=4; *p<0.001 with respect to LPS, **p<0.001 with respect to PGB or HA alone.

24-hour exposure of HCECs to 500 ng/mL LPS induced maximal secretion of IL-1β, IL-6, and TNF-α, all of which are released in small amounts under basal conditions, as shown in Figure 22.

The lipopolysaccharide strongly increased cytokine secretion. Preincubation (1h) with 100 µg/m PGB or 100 µg/ml HA significantly reduced LPS-stimulated cytokine secretion.

After 1 hour of incubation of a composition with the combination (mixture) of PGB+HA in a 1/1 ratio, the secretion of IL-1β, IL-6 and TNF-α has significantly reduced, demonstrating a synergistic effect of the combination of the two compounds in inhibiting cytokine production.

Finally, among the cytokines examined, we explored IL6, which was the most inhibited, in three selected ratios between the two compounds (PGB/HA 1/1, 1/2 and 1/3), demonstrating that synergistic activity is preserved in the selected ratios examined (Figure 22).

### Statistical analysis

The data are expressed as the mean ± standard error of the mean (SEM) of three independent experiments. Statistical significance was determined using one-way analysis of variance (ANOVA), followed by Tukey's post hoc test. A p-value < 0.05 was considered statistically significant.

### Conclusions

The test results show how the combination of the active ingredients pregabalin (PGB) + hyaluronic acid (HA) in the range from 1 to 3 (PGB 1 : HA 1, 2, 3) inhibits the activity of Nf-kβ and reduces the expression of pro-inflammatory cytokines in an in vitro model of corneal inflammation, demonstrating a synergistic action preferably in the PGB/HA ratio range from 1 : 1 to 1 : 3.

The disclosures in Italian Patent Application No. 102024000001167, from which this application claims priority, are incorporated herein by reference.

## Claims

1. An ophthalmic composition comprising hyaluronic acid optionally in salt form in combination with pregabalin and esters and salts thereof in an amount that is therapeutically effective in modulating pain transmission, and a physiologically acceptable water-based carrier for use in the prevention and/or treatment of inflammation disorders of the surface of the eye, and/or, wherein pregabalin and hyaluronic acid are in a ratio of 1:10 to 3:1, preferably 1:1 to 1:3.

2. The composition for use according to claim 1, wherein the inflammation disorders are dry eye, keratoconjunctivitis sicca.

3. The composition for use according to claim 1 or 2, wherein the hyaluronic acid is in a free, non-cross-linked form, optionally in a form salified with a salt, or is in a cross-linked form with a physiologically acceptable cross-linking agent.

4. The composition for use according to any one of claims 1-3, wherein pregabalin is in a concentration from 0.05% to 0.2% and the hyaluronic acid is in a concentration of 0.1 to 0.6% by weight.

5. The composition for use according to any one of claims 1-4, wherein the hyaluronic acid optionally in salt form has an average molecular weight greater than 300,000 Da, preferably comprised between 1,000,000 and 6,000,000, between 1,500,000 and 3,500,000 Daltons.

6. The composition for use according to any one of claims 1-5, wherein the pH is comprised between 6.4 and 7.6.

7. The composition for use according to any one of claims 1-6, further comprising:
- a buffer or buffer system for adjusting the pH value preferably selected from sodium hydrogen phosphate, disodium phosphate, sodium citrate, sodium borate/boric acid, and mixtures thereof, and/or
- an isotonizing agent selected from sodium chloride, potassium chloride, magnesium chloride, and mixtures thereof.

8. The composition for use according to any one of claims 1-7, wherein the treatment of dry eye and/or keratoconjunctivitis sicca is chronic.

9. The composition for use according to any one of claims 1-8 in the form of drops, eye drops, gel, or artificial tears for instillation into the eye, preferably formulated as single-dose or multi-dose dosage unit, preferably preservatives free.

10. The ophthalmic composition comprising pregabalin and salts and esters thereof and hyaluronic acid optionally in salt form and a physiologically acceptable carrier in liquid or gel form for simultaneous, separate or sequential use with one or more ophthalmic medications for the treatment of discomfort or pain associated with multiple and/or simultaneous instillation of said ophthalmic medications.

11. The ophthalmic composition for use according to claim 10, wherein pregabalin and hyaluronic acid are in a ratio of 1:10 to 3:1, preferably 1:1 to 1:3.

12. The ophthalmic composition for use according to claim 10 or 11, wherein said ophthalmic medications are ophthalmic preparations for topical use, preferably eye drops, containing an active ingredient with anti-inflammatory activity, preferably NSAID or steroid.

13. The ophthalmic composition for use according to any one of claims 10-12 in the treatment of a population of individuals affected by an inflammatory disease that is systemic or local to the eyes.

14. The ophthalmic composition for use according to any one of claims 10-13, wherein said discomfort is a stinging or diffuse pain of the eye.

15. An ophthalmic composition comprising pregabalin and salts and esters thereof and hyaluronic acid, optionally in salt form, and a physiologically acceptable carrier in liquid or gel form for use in the treatment of an inflammatory disease of the eye.
